# EUROPEAN PATENT APPLICATION

(11) **EP 4 201 305 A1**
(43) Date of publication of application: **28.06.2023**
(21) Application number: 21216910.6
(22) Date of filing: 22.12.2021
(51) Int. Cl.: A61B 5/00, A61B 5/024, A61B 5/145, A61B 5/1455, A61B 5/1464, A61B 5/01, A61B 5/0205

(54) **A FETAL MONITORING DEVICE**

(71) Applicant: University College Cork, National University Of Ireland, Cork (IE)
(72) Inventor: BURKE, Ray, Cork (IE); MCCARTHY, Fergus, Cork (IE)
(74) Representative: FRKelly

(57) **Abstract**

The present invention provides a fetal monitoring device, and in particular a real time fetal pH, lactate and physiological monitoring device for detection and prevention of perinatal hypoxia, the device including an optical sensor operable to emit light at a wavelength of between 1000nm-3000nm onto vascularised tissue on the fetal scalp and to detect the reflected light, in addition to a processor operable to convert real time spectroscopy data from the reflected light into correlated real time data regarding one or more biological markers.

## Description

### Field of the invention

This invention relates to a fetal monitoring device, and in particular a real time fetal pH, lactate and physiological monitoring device for detection and prevention of perinatal hypoxia.

### Background of the invention

Perinatal hypoxia or asphyxia during labour is the partial or complete lack of oxygen to neonates at the time of labour and delivery. This devastating complication of childbirth is associated with up to 60% of babies dying and represents a significant unmet clinical need in maternal and infant care. Intra-partum hypoxia can lead to several serious medical conditions affecting the neurological system, resulting in cases such as Hypoxic Ischemic Encephalopathy (HIE) and other brain injuries that may lead to paralysis and severe brain damage causing debilitating cognitive delays, developmental problems, and motor impairments.

All babies born by vaginal delivery are potentially at risk of hypoxic injury. Suspected hypoxic injury assessed by current state of the art heart rate monitoring instruments, has been a long-standing and common problem managed in labour wards by obstetricians and midwives globally.

Current methods of fetal monitoring during labour include cardiotocography (CTG; fetal heart rate monitoring), the current gold standard and Fetal Blood Sampling (FBS). These technologies are limited by technical challenges such as the necessity for advanced cervical dilation, requirement for regional analgesia or volume of blood required to ascertain fetal pH. Use of computerised interpretation of CTG in women who have continuous electronic fetal monitoring in labour has also been shown to not improve clinical outcomes for mothers or babies (Brocklehurst et al, Lancet 2017). As a result, inadvertent caesarean sections are performed due to false concerns around CTG readings. Furthermore, misinterpretation, or inability to interpret the CTG, resulting in hypoxic brain injury is a cause of litigation exceeding 1 billion pounds in the UK annually.

A fetal scalp lactate measurement of ≥4.8 mmol/L has a positive predictive value (PPV) of 1% and a negative predictive value (NPV) of 100% in predicting umbilical artery pH≤7.00, and a PPV of 5% and a NPV of 98% in predicting umbilical artery pH≤7.10. The sensitivity and specificity for these values were 100%, 23% and 90%, 23% respectively. The ascertainment of continuous fetal blood samples during labour using current instrumentation is not safe or feasible. Currently the only method to obtain intrapartum fetal pH/ lactate is via fetal blood sampling which involves a small laceration using a 2mm long blade to obtain a 100µl blood sample from the fetal capillary bed. There is therefore a need for an improved means of detecting and therefore preventing fetal hypoxic brain injury. Accurate detection of fetal hypoxia will ensure that caesarean sections are performed only when absolutely required. This contrasts with the current situation where reliance on fetal heart rate monitoring which has poor sensitivity and specificity for fetal hypoxia results in high levels of Caesarean section incorrectly performed for suspected fetal distress.

Given that 75% of labour cases occur gradually, preventative monitoring is highly desirable at this point in the patient pathway and can result in positive patient outcomes, an improved level of maternity care and a long-term reduction in patient care costs in both mother and child.

It is therefore an object of the present invention to provide a fetal monitoring device which improves the degree of accuracy and reliability of fetal monitoring when compared to the current gold standard.

### Summary of the invention

According to a first aspect of the present invention there is provided a fetal monitoring device comprising a substrate adapted for in-utero adhesion to vascularised tissue on the head of a fetus; at least one optical sensor mounted to the substrate and operable to emit light at a wavelength of between 1000nm-3000nm onto the vascularised tissue and to detect the reflected light; and a processor operable to convert real time spectroscopy data from the reflected light into correlated real time data regarding one or more biological markers to enable an assessment of fetal wellbeing during labour.

Preferably, the optical sensor is operable to emit light at a wavelength of between 1350nm-2500nm.

Preferably, the optical sensor comprises at least one multispectral light source.

Preferably, the optical sensor is operable to simultaneously and/or sequentially emit and detect light at different wavelengths and/or wavelength bands.

Preferably, the optical sensor is operable to emit light in two or more wavelength bands selected from 1450+/-50nm, 2100+/-50nm, 2300+/-50nm and 2500+/-50nm.

Preferably, the processor is operable to convert real time spectroscopy data from the reflected light into measurements of one or more of blood lactate level, blood pH level, blood oxygen saturation, blood carbon dioxide level, body temperature, hydration and heart rate.

Preferably, the optical sensor comprises a plurality of optical emitters.

Preferably, the optical sensor comprises a plurality of optical detectors.

Preferably, at least one of the optical detectors comprises a reflectance based sensor.

Preferably, the optical sensor is adapted to achieve an optical depth of penetration into the vascularised tissue of less than 5mm, more preferably less than 3mm and most preferably less than 2mm.

Preferably, the monitoring device comprises a discrete temperature sensor.

Preferably, the monitoring device comprises a discrete pH sensor.

Preferably, the monitoring device comprises two or more electrodes operable to convey an electrical signal through the vascularised tissue.

Preferably, the processor is operable to utilise data from the two or more electrodes to enable the processor to perform electrochemical impedance spectroscopy.

Preferably, the processor utilises an algorithm to convert the real time spectroscopy data into the correlated real time data regarding the one or more biological markers.

Preferably, the substrate comprises a biocompatible adhesive for securing the substrate to the vascularised tissue.

Preferably, the monitoring device comprises a vacuum cup for securing the substrate to the vascularised tissue.

Preferably, the monitoring device comprises a probe defining the substrate and a remote processing unit comprising the processor.

Preferably, the monitoring device comprises a tether connecting the probe and the processing unit.

Preferably, the tether defines an electrical, optical and/or fluid communication path between the probe and the processing unit.

According to a second aspect of the present invention there is provided a method of in-utero monitoring of fetal levels of selected biomarkers, the method comprising the steps of emitting light at a wavelength of between 1 000nm-3000nm onto vascularised tissue on the head of a fetus; and converting real time spectroscopy data from the reflected light into correlated data regarding one or more biological markers.

Preferably, the method comprises emitting light onto the vascularised tissue in two or more wavelength bands selected from 1450+/-50nm, 2100+/-50nm, 2300+/-50nm and 2500+/-50nm.

Preferably, the method comprises converting the real time spectroscopy data from the reflected light into measurements of one or more of blood lactate level, blood pH level, blood oxygen saturation, blood carbon dioxide level, body temperature, hydration and heart rate.

### Brief description of the drawings

The present invention will now be described with reference to the accompanying drawings, in which:
Figure 1 illustrates a perspective view of a fetal monitoring device according to a preferred embodiment of the present invention;
Figure 2 illustrates a side elevation of the fetal monitoring device shown in Figure 1;
Figure 3 illustrates a top plan view of the device of Figures 1 and 2;
Figure 4 illustrates a bottom plan view of the device of Figures 1 to 3;
Figure 5 illustrates a sectioned side elevation of the device of Figures 1 to 4;
Figure 6 illustrates a perspective view of a portion of a tether forming part of the device shown in Figures 1 to 5;
Figure 7 illustrates experimental data plotting the reflectance spectrum of human blood against wavelength of incident light for a range of pH values, demonstrating that reflectance is sensitive to pH changes in the blood;
Figure 8 illustrates a schematic representation of an exemplary algorithm utilised by the fetal monitoring device of the invention in the real time processing of acquired data;
Figure 9 illustrates an exploded perspective view of an alternative embodiment of a fetal monitoring device according to the present invention;
Figure 10 illustrates the exploded perspective view of Figure 9 from below; and
Figure 11 illustrates a sectioned side elevation of the monitoring device of Figures 9 and 10.

### Detailed description of the drawings

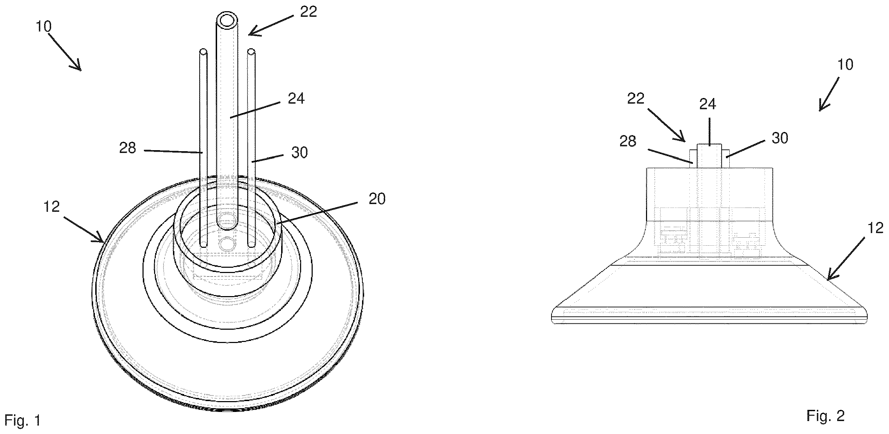

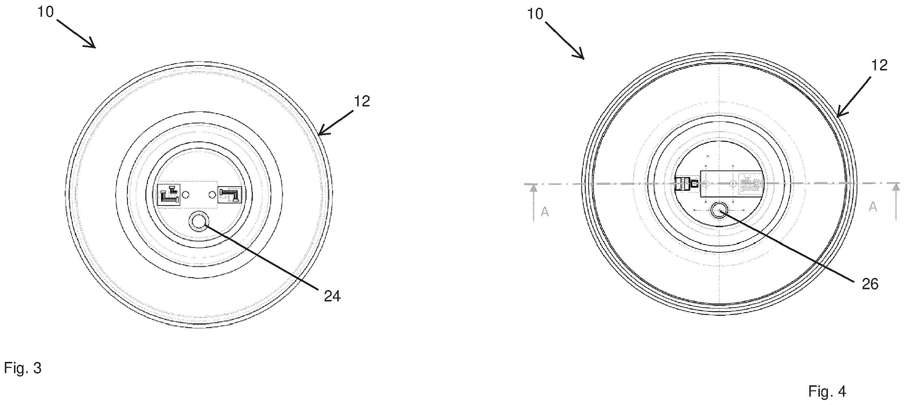

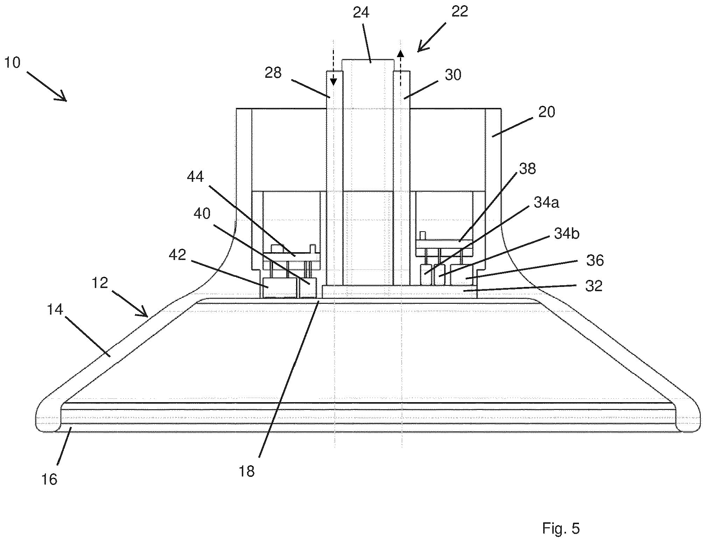

Referring now to Figures 1 to 6 of the accompanying drawings there is illustrates a first embodiment of a fetal monitoring device, generally indicated as 10, for use in real time monitoring of fetal bio-signs, in particular blood lactate and pH in order to monitor the health of the fetus in particular in relation to hypoxia during labour.

As explained in detail hereinafter, the monitoring device 10 operates using near-infrared spectroscopy (NIRS) and is placed on the fetal scalp during labour at the time of routine vaginal examination by a health professional to provide a real-time readout of lactate/pH levels to provide visibility and thus reassurance to physicians and midwives on fetal status. Using advances in NIRS technology focused on specific spectral wavelength bands linked to key biomarkers the monitoring device 10 can continuously measure key parameters, primarily blood lactate level and blood pH level, but optionally also blood oxygen saturation, blood carbon dioxide level, body temperature, hydration and heart rate. The monitoring device 10 is also capable of multimodal operation in order to additionally monitor temperature, heat flow, pCO₂ (partial pressure of carbon dioxide), and pO₂ (partial pressure of oxygen) which data can be used to augment the measurement of the principle components or key measured parameters of lactate and pH to facilitate improved data processing by a novel algorithm executable on a processor (not shown) forming part of the monitoring device 10 to thereby provide supporting data to the clinicians.

As noted above, a fetal scalp lactate measurement of ≥4.8 mmol/L had a positive predictive value (PPV) of 1% and a negative predictive value (NPV) of 100% in predicting umbilical artery pH ≤7.00, and a PPV of 5% and a NPV of 98% in predicting umbilical artery pH ≤7.10. The sensitivity and specificity for these values were 100%, 23% and 90%, 23%, respectively. The monitoring device 10 of the present invention is based on non-invasive assessment of the lactate and/or pH levels within this capillary bed at a depth of approximately 2mm under the fetal skin. It is clinically universally accepted that this lactate/pH measurement reflects fetal systemic acid base balance and predicts adverse neonatal outcomes.

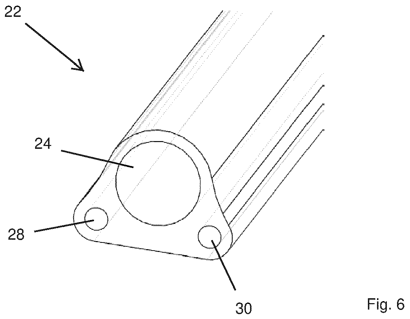

The monitoring device 10 utilises this clinical premise but accesses the capillary bed non-invasively via NIRS. The monitoring device 10 thus comprises a probe defined by a suction cup 12 which is preferably formed from a biocompatible and resiliently deformable material, and shaped and dimensioned to be attached to the fetal scalp in the fontanelle region without piercing the epidermis of the fetus. The suction cup 12 comprises a conical sidewall 14, an open mouth 16 and an opposed base 18 which defines a sensor surface or substrate via which various sensors, as detailed hereinafter, can communicate with the fetal scalp in order to measure the bio-markers and/or other parameters. The suction cup 12 further comprises a neck 20 in which the various sensors are housed, and to which is connected a tether 22, a portion of which is shown in isolation in Figure 6. The tether 22 allows the monitoring device 10 to communicate with one or more external systems for data processing, light delivery and detection, vacuum generation, power, etc., but also to provide an externally accessible physical connection to allow the monitoring device 10 to be quickly and easily retrieved if required. However, as described in detail in relation to a later embodiment, the monitoring device of the present invention may also be provided in an untethered form between which communication may be achieved wirelessly.

The tether 22 defines a communication path to the suction cup 12, and for example defines a vacuum lumen 24 which terminates at an opening 26 in the base 18, allowing the vacuum lumen 24 to communicate with the interior space defined between the suction cup 12 and the fetal scalp. The vacuum lumen 24 can be used to apply a vacuum within this space to adhered the suction cup 12 to the scalp, preferably with the base 18 drawn downwardly into close proximity or contact with the fetal scalp in order to improve the efficacy of the one or more sensors described in detail hereinafter. One or more of said sensors may be used to monitor for contact of the base 18 with the scalp in order to control the level of vacuum applied in order to securely but safely maintain adherence of the suction cup 12 to the scalp.

In addition to the above functionality the vacuum lumen 24 may be used to affect a flushing function in order to clear the space defined between the suction cup 12 and the scalp before the suction cup 12 is adhered in place. This may be achieved by using a reduced negative pressure sufficient to remove the presence of any interference such as biological fluids or other matter which might adversely affect the operation or accuracy of the sensors. Additionally or alternatively a flushing liquid or gas may be pumped down the vacuum lumen 24 into the space between the suction cup 12 and scalp, and subsequently withdrawn through the vacuum lumen 24, or an additional exhaust lumen (not shown) which may be provided, in order to remove the flushing liquid and entrained contaminants. Once the flushing action has been completed the negative pressure applied through the vacuum lumen 24 may be suitably increased to affect adherence of the suction cup 12 to the scalp. It will be appreciated that the negative pressure may be applied via any suitable means (not shown), and in the present embodiment from a location remote from the monitoring device 10. A biocompatible adhesive (not shown) may also be provided on the interior surface of the suction cup 12, or around the mouth 16, in order to further improve adhesion to the fetal scalp.

The tether 22 further defines part of an optical sensor of the monitoring device 10 and comprising an emitter in the form of a first light guide 28 via which light may be delivered to the base 18 of the section cup 12, and a detector comprising a second light guide 30 for retrieving the light reflected from the fetal scalp, for external/downstream measurement and processing. For example the second light guide 30 can transfer the reflected light from the tissue to a spectrometer (not shown) or detectors located in a hand held unit (not shown) or a typical medical device console unit (not shown). The reflected light can be processed by the spectrometer to provide the corresponding blood lactate and/or pH level, being key biomarkers, to reflect fetal systemic acid base balance and predict adverse neonatal outcomes, as detailed hereinafter. The processor running the algorithm to analyse the spectral data may be part of the above mentioned hand held unit or medical device console, but it is also envisaged that the processor may be housed locally at the suction cup 12.

In the embodiment illustrated the first and second light guides 28, 30 each comprise one or more NIR glass fibres which terminate at a NIR transparent cover 32, preferably glass, mounted in the base 18 and allowing light to pass from the first light guide 28 into the interior of the suction cup 12 to be incident on the scalp, and to pass back into the second light guide 30 to be transmitted to the above mentioned externally located optical spectrometer (not shown) or alternative processing equipment by which the spectrum of the reflected light can be analysed. The use of glass fibres for the first and second light guides 28, 30 provides fibre optic coupling between both the light source and the target site on the fetal scalp and for the reflected light to be returned to the spectrometer or other detector (not shown). This ensures a robust and reliable optical and physical connection between the suction cup 12 and the external hardware via which the spectrum is processed for interpretation by the medical personnel present.

An external light source (not shown) is connected or otherwise associated with a distal end (not shown) of the first light guide 28, and may for example comprise an LED, SLED, laser or other suitable light source to thus form part of the optical sensor. The external light source is preferably configured to emit light at a wavelength of 1100-2300nm in low doses of energy to ensure biologically safe operation. In particular the external light source is operable to emit light in two or more wavelength bands selected from 1450+/-50nm, 2100+/-50nm, 2300+/-50nm and 2500+/-50nm. The emitted light is used to non-invasively sense the lactate concentration and the pH (biomarkers) in the vascularised tissue on the forehead to a depth limited by the transmission of light through tissue in the NIR, for example to a maximum of 2 or 3mm depth. Near Infrared Spectroscopy (NIRS) thus forms the primary measurement mode of the monitoring device 10 using light in the near infrared spectrum passed through the tissue in reflectance mode.

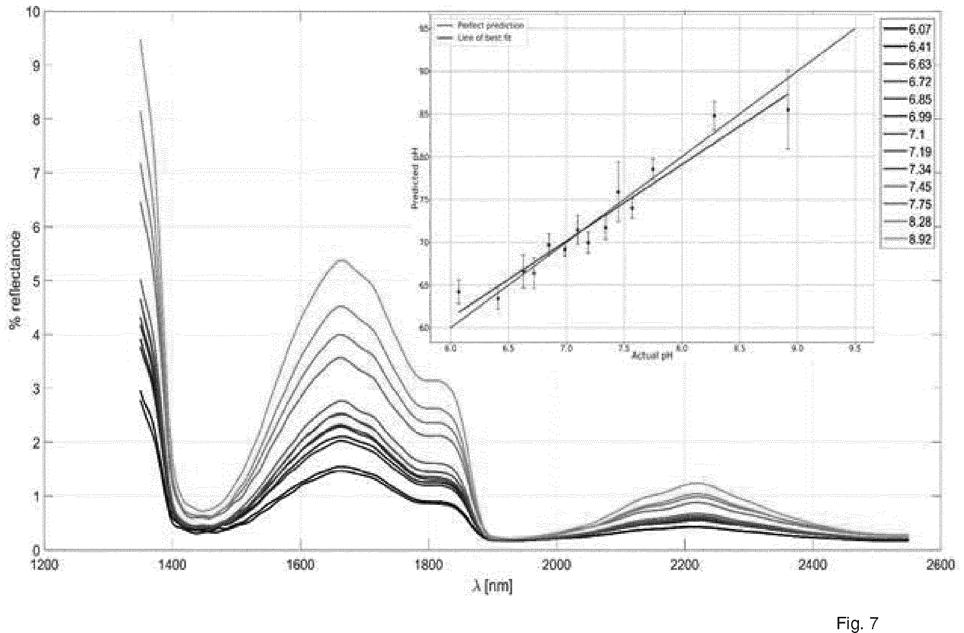

The NIRS measurement is employed to generate a spectrum that can be measured and analysed for lactate and pH components or biomarkers. For lactate measurement the light from the external (or local) source passes through the fibres of the first light guide 28 into the fetal scalp tissue. The light diffuses through the tissue and is collected via the fibres of the second light guide 30 which is connected to the above mentioned spectrometer which produces a spectrum in the required wavelength range. The spectrometer measurement is calibrated by doing a background measurement initially with no light source (dark measurement) then a measurement is taken with a known reference material. The sample measurement is then taken and the background and reference measurement are compared or computed with the measurement to extract the signal for lactate and pH. Data normalisation for example to the dominant absorption peaks of water or some other reference spectra may also be undertaken as part of the signal extraction and data analysis. For example, referring to Figure 7 there is illustrated experimental data plotting the reflectance spectrum of human against wavelength of light for various pH values. The percentage reflectance (from 0% to 10%) at wavelengths from 1350nm to 2500nm for different pH levels is shown. A peak at approximately 1680nm indicates a change in spectrum to the corresponding change in pH. The inset in Figure 7 shows the variation in actual pH to the measured or predicted pH at 1680nm. This form of data can be accessible to and utilised by the data processing algorithm utilised by the monitoring device 10 as a reference against which real time reflectance is compared as part of a larger data processing procedure in order to present meaningful information to the medical personal regarding the status of the fetus, an example of which is detailed hereinafter.

The monitoring device 10 further comprises an additional discrete optical sensor comprising a first emitter 34a, a second emitter 34b, and a detector 36, all of which are mounted in optical contact with the glass cover 32 to provide a protective covering thereto, and directed into the space defined by the suction cup 12. The emitters 34a, 34b and detector 36 are mounted to an adjacent circuit board 38 secured within the neck 20 of the suction cup 12. The first and second emitters 34a, 34b are operable to emit light at a particular wavelength, preferably in the infrared and red bands of the electromagnetic spectrum, and may comprise any suitable light source such as an LED, SLED or laser. The first and second emitters 34a, 34b preferably emit light at different wavelengths, and may be operated sequentially or simultaneously. However it is also envisaged that a single emitter could be employed that is operable to emit light of different wavelengths, for example a supercontinuum source. One of the first and second emitters 34a, 34b preferably emits light below 800nm, typically 600nm and the other above 800nm, typically 900nm. A third emitter (not shown) may be employed, and for example emitting light such as 800nm to improve the accuracy of the measurement. The emitters are preferably spaced from one another, and where a third emitter is utilized, the three emitters may be arranged in a triangular arrangement. The detector 36 is operable to measure light reflected off the fetal scalp from the first and second emitters 34a, 34b.

This discrete optical sensor is particularly configured to perform real time measurement of SPO₂, also known as reflectance pulse oximetry, in addition to pulsatile heartrate monitoring based on the volumetric change of blood in the measurement area. This optical sensor may be operated sequentially or simultaneously with the optical spectrometry sensor described above. The data obtained regarding SPO₂, and/or heartrate can then be utilised by the data processing algorithm employed by the monitoring device 10 to improve the accuracy of the real time clinical diagnosis.

All of the light sources utilised in the monitoring device 10 preferably operate at low power levels for patient and eye safety. The sources may be pulsed light sources to support power management of as part of a data processing strategy.

The monitoring device 10 additionally comprises a temperature sensor 40 which may be a thermistor or any other suitable component or assembly of components and which may be arranged to be in close or direct contact, in use, with the fetal scalp. The temperature sensor 40 may additionally or alternatively be configured for monitoring heat flow (area temperature measurement). As an alternative to a thermistor the temperature sensor 40 may comprise for example a Mid Infrared (MIR) non contact sensor to measure temperature. An accurate measurement of temperature for the fetus is an important diagnostic parameter which is not currently measured in real time but when combined with pH and lactate may enhance the real time clinical assessment of fetal well-being. High temperatures (pyrexia) during labour may aggravate an existing hypoxia due to increased tissue oxygen consumption and a shift of the oxygen dissociation curve to the right. Furthermore, the risk of cerebral palsy increases in children born by mothers with clinical signs of chorioamnionitis and fever. Adjacent to the the temperature sensor 40 is an impedance/capacitance/resistance sensor 42 which may for example comprise a pair of discrete electrodes (not shown) of metal or other suitable material, and which may again be arranged to be in close or direct contact, in use, with the fetal scalp. The sensor 42 is preferably operable to use the electrodes (not shown) to detect tissue water content or hydration in the fetal scalp by means of electrochemical impedance spectroscopy (EIS) but is not limited to measuring this parameter. The electrodes may be electrically insulated or contact metal electrodes. The electrodes may also be utilized to detect biomarkers and/or tissue types and to determine correct placement of the suction cup 12 to ensure suitable and accurate placement of the various sensors therein.

The tether 22 thus also provides a path for the supply of power to the above microelectronic sensors, and a path for electronic communication therewith, including the emitters 34a, 34b, detector 36, temperature sensor 40 and impedance sensor 42. Again it will be appreciated that it is envisaged that an untethered variant of the monitoring device may be employed, and which would therefore be provided with a local power supply for such components.

The monitoring device 10 may also employ optical and/or electrical means (not shown) to heat the fetal scalp tissue locally to a maximum of 45°C to enhance blood perfusion and therefore the above described measurement techniques for better detection.

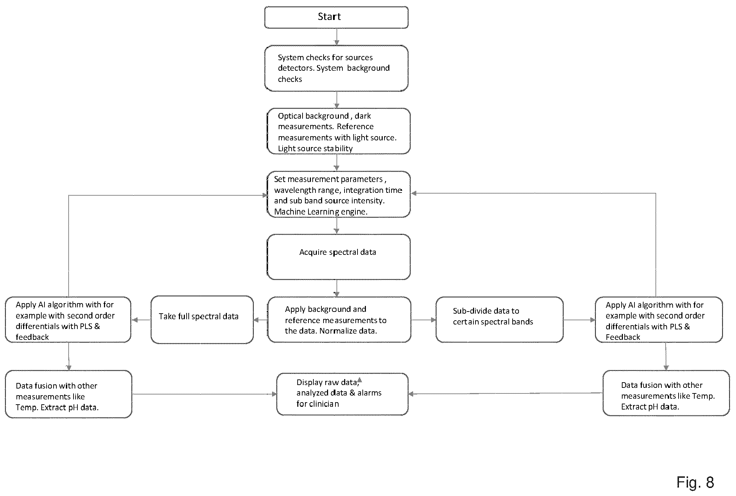

In use the monitoring device 10 is secured to the fontanelle region of the fetal scalp in utero, as hereinbefore described. A flushing process may be employed to improve sensor operation, and the suction cup 12 then suitably secured in place by the application of a light vacuum and/or biocompatible adhesive. Referring then to Figure 8 the monitoring device 10 may perform system checks for the various light sources, detectors and other sensors to ensure these components are operational. Any other necessary background checks may be performed at this stage. The optical sensors may then be activated in order to establish an optical background or base measurement and to take dark measurements and/or other reference measurements and to establish light source stability.

The next step in the operation of the monitoring device 10 is to set the various measurement parameters for each of the sensors, for example wavelength range of emitted light, integration time and sub band source intensity, temperature or impedance measurement intervals, and the machine learning engine parameters if employed as part of the algorithm. The integration time is the time taken to acquire a spectra. In low light conditions a long integration time of 200 mS can be used to accumulate more photons hence more signal. In a case of high signal intensity a low integration time for example 10mS could be used. The integration time can be programmed on the spectrometer and this parameter may be adjusted as part of a detection algorithm.

The monitoring device 10 will then be operated to emit light onto the fetal scalp from the first light guide 28 and to transmit the reflected light, via the second light guide 30, to the spectrometer (not shown). The spectrometer will then acquire the spectral data for the reflected light. Preferably sequentially but potentially simultaneously the discrete optical sensor including the first and second emitters 34a, 34b and detector 36 are actuated to take regular additional measurements such as SPO₂ levels and heartrate.

The data processing algorithm will then apply the requisite background and/or reference measurements to the data, for example factoring in the influence of the water spectra based on the water content of the irradiated tissue, which may for example be determined by the Impedimetric sensor 42. The tissue water content and absorption at the NIR wavelengths and a water spectrum are therefore utilised by the algorithm as a reference spectrum along with the lipid spectrum and the standard background and correction for the source/emitter. As the water absorption spectrum dominates in the NIR the signal needs to be continuously referenced to the absorption data for water. Certain parts of the water spectrum may be used by the data processing algorithm as a reference for a ratio metric signal generation at multiple wavelengths. The signal processing algorithm may also include correlated and auto correlated sampling techniques, machine learning and Al.

The algorithm may then examine the full spectral data and/or divide the data into certain spectral bands, and further process the data, for example using second order differentials, with Partial Least Squares Regression (PLS-R). The aim of PLS-R is to build a linear model of a set of response variables (Lactate or pH values) and the predictor matrix (reflectance spectra). PLS-R projects the data to a reduced dimension matrix space but also projects the data labels to this matrix space, allowing for a regression model to be built. PLS is a linear method that extracts a linear model from the data. Due to the high dimensionality of NIR spectra, PLS is the most widely used tool for modelling the spectral data. However, other algorithms for building non-linear models such as artificial neural networks (ANN) or support vector regression (SVR) may be applied as a part of the algorithmic processing of the raw data. A dimension reduction or feature selection method like PCA should be used before applying these algorithms.

The processed data may be utilised in a feedback loop to further refine the measurement parameters, wavelength ranges, etc. In addition the data may be further processed or combined with additional data obtained by the sensors of the monitoring device 10, for example temperature, impedance and pH data, etc. This additional data may be applied to further refine the data output from the algorithm, which can then be displayed in an appropriate format to the clinicians. For example the raw data, analyzed data and/or alarms may be displayed in real time. Such alarms may be indicative of various bio-signs such as hypoxia.

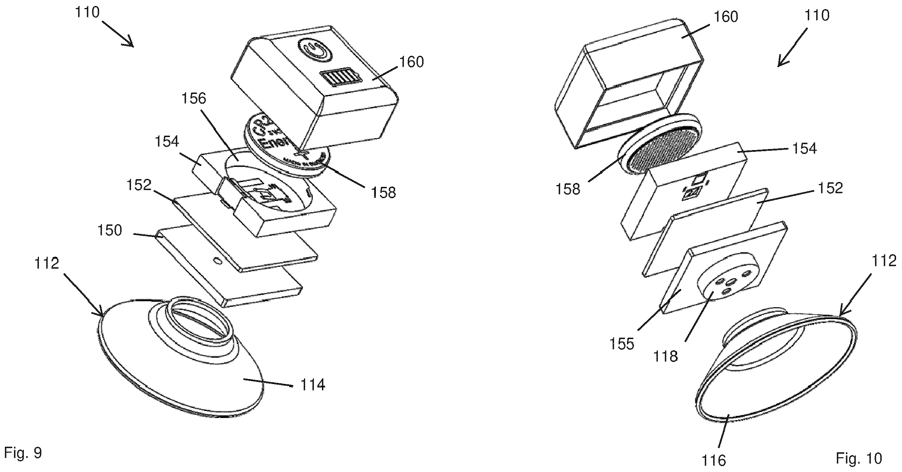

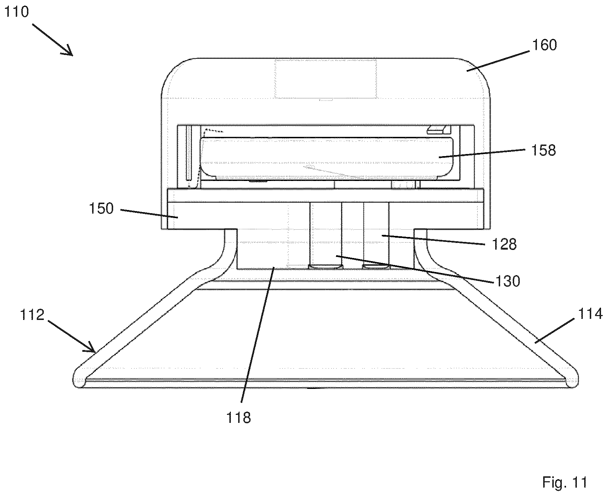

Referring now to Figures 10 and 11 there is illustrated an alternative embodiment of a fetal monitoring device according to the present invention, generally indicated as 110. In this alternative embodiment like components have been accorded like reference numeral and unless otherwise stated perform a like function. The most notable difference with the monitoring device 110 is the ability to operate untethered and to communicate with an external module, handheld unit or the like (not shown) wirelessly in order to transmit either raw or processed data as detailed hereinafter.

The monitoring device 110 comprises a suction cup 112 having a substantially conical sidewall 114, a mouth 116 to be applied to the fetal scalp, and a closed base 118 defined as part of a sensor substrate 150 on which one or more optical and other sensors may be located for communication with the fetal scalp during use. These sensors are functionally equivalent to the spectrometry sensors contained in the monitoring device 10, for example one or more optical sensors capable of emitting and detecting light at particular wavelength bands , in particular an emitter 128 and detector 130, temperature sensors (not shown), impedance sensors (not shown), etc. The monitoring device 110 thus comprises a printed circuit board forming the sensor substrate 150 and on which are mounted the various microelectronic sensors. An additional electronics substrate 152 is provided adjacent the sensor substrate 150, carrying the requisite electronics to operate the sensors and thus provide a physical interface with the sensors.

A power electronics module 154 is located adjacent the electronics substrate, and preferably includes a battery tray 156 for receiving a suitable battery 158 to provide discrete power to the monitoring device 110. A housing 160 encloses the various electronic components and maintains a suitable seal during use.

In use the monitoring device 110 operates in substantially the same manner as the tethered monitoring device 10, but does not receive light from a remote source or return the reflected light to a remote spectrometer. As a result the wireless monitoring device 110 contains the discrete light emitter 128 and paired detector 130, although additional optical sensors may be employed. The device 110 may contain a microelectronic spectrometer (not shown), for example a C14272, C13272 and/or C14273 MEMS-FPI spectrum sensor as manufactured by Hamamatsu in order to facilitate the local measurement of the reflected light spectra, and may also include a processor (not shown) on which the above described data processing algorithm may be executed. However it is also intended that the reflected light data may be wirelessly transmitted from the monitoring device 110 for remote processing.

The monitoring device 110 may also comprise means for generating a negative pressure in order to adhere the device 110 to the fetal scalp, but is more likely due to space constraints to utilise mechanical generation of a negative pressure by the physical compression of the suction cup 112 against the scalp during initial application of the device 110. In either case a biocompatible adhesive may additionally or alternatively be employed as hereinbefore described.

The present invention thus provides a multimodal monitoring device which adheres to the fetal scalp during labour via gentle vacuum assisted suction and/or adhesive for the duration of labour, and which is therefore simple to apply and which can be easily removed by the clinician following delivery. The use of optical sensors and a novel data processing the algorithm the monitoring device can extract, in real time, pH and lactate signals from the spectra. The monitoring device of the invention therefore allows for continuous measuring of foetal pH and lactate during labour to provide clinicians with a significantly improved, non invasive means by which to monitor the fetus for issues such as hypoxia.

## Claims

1. A fetal monitoring device comprising a substrate adapted for in-utero adhesion to vascularised tissue on the head of a fetus; at least one optical sensor mounted to the substrate and operable to emit light at a wavelength of between 1000nm-3000nm onto the vascularised tissue and to detect the reflected light; and a processor operable to convert real time spectroscopy data from the reflected light into correlated real time data regarding one or more biological markers to enable an assessment of fetal wellbeing during labour.

2. A fetal monitoring device according to claim 1 in which the optical sensor is operable to emit light at a wavelength of between 1350nm-2500nm.

3. A fetal monitoring device according to claim 1 or 2 in which the optical sensor comprises at least one multispectral light source.

4. A fetal monitoring device according to any preceding claim in which the optical sensor is operable to simultaneously and/or sequentially emit and detect light at different wavelengths and/or wavelength bands.

5. A fetal monitoring device according to claim 4 in which the optical sensor is operable to emit light in two or more wavelength bands selected from 1450+/-50nm, 2100+/-50nm, 2300+/- 50nm and 2500+/-50nm.

6. A fetal monitoring device according to any preceding claim in which the processor is operable to convert real time spectroscopy data from the reflected light into measurements of one or more of blood lactate level, blood pH level, blood oxygen saturation, blood carbon dioxide level, body temperature, hydration and heart rate.

7. A fetal monitoring device according to any preceding claim in which the optical sensor comprises a plurality of optical emitters.

8. A fetal monitoring device according to any preceding claim in which the optical sensor comprises a plurality of optical detectors.

9. A fetal monitoring device according to claim 8 in which at least one of the optical detectors comprises a spectrometer.

10. A fetal monitoring device according to claim 8 or 9 in which at least one of the optical detectors comprises a reflectance based sensor.

11. A fetal monitoring device according to any preceding claim comprising a discrete temperature sensor.

12. A fetal monitoring device according to any preceding claim comprising a discrete pH sensor.

13. A fetal monitoring device according to any preceding claim comprising two or more electrodes operable to convey an electrical signal through the vascularised tissue.

14. A fetal monitoring device according to any preceding claim in which the processor utilises an algorithm to convert the real time spectroscopy data into the correlated real time data regarding the one or more biological markers.

15. A fetal monitoring device according to any preceding claim comprising a vacuum cup for securing the substrate to the vascularised tissue.
